# EUROPÄISCHE PATENTANMELDUNG

(11) **EP 2 995 600 A1**
(43) Veröffentlichungstag der Anmeldung: **16.03.2016**
(21) Anmeldenummer: 14184458.9
(22) Anmeldetag: 11.09.2014
(51) Int. Cl.: C07C 5/13

(54) **Verfahren und Anlage zur Herstellung von Kohlenwasserstoffen**

(71) Anmelder: Linde Aktiengesellschaft, 80331 München (DE)
(72) Erfinder: Fritz, Helmut, 81375 München (DE); Höfel, Torben, 81543 München (DE)
(74) Vertreter: m patent group

(57) **Zusammenfassung**

Es wird ein Verfahren zur Herstellung von Kohlenwasserstoffen vorgeschlagen, das umfasst, in einer Katalyseeinheit (1) unter Verwendung eines oder mehrerer Oxygenate und/oder Olefine enthaltender Katalyseeinsatzströme (a) einen n-Butan, Isobutan, 1-Buten, 2-Buten, Isobuten und Kohlenwasserstoffe mit mehr als vier und/oder weniger als drei Kohlenstoffatomen enthaltenden Katalyseproduktstrom (b) zu erzeugen, und das ferner umfasst, in einer Dampfspalteinheit (2) unter Verwendung eines oder mehrerer Dampfspalteinsatzströme (g, n, l, r) einen Dampfspaltproduktstrom (s) zu erzeugen. Es ist vorgesehen, dass aus dem Katalyseproduktstrom (b) oder einem Teil hiervon zumindest der überwiegende Teil der Kohlenwasserstoffe mit mehr als vier und/oder weniger als drei Kohlenstoffatomen und des Isobutens entfernt wird und ein oder mehrere jeweils verbleibende Ströme (g, n, r) als der oder die Dampfspalteinsatzströme (g, n, l, r) verwendet werden. Eine entsprechende Anlage (100, 200, 300) ist ebenfalls Gegenstand der Erfindung.

## Beschreibung

Die Erfindung betrifft ein Verfahren und eine Anlage zur Herstellung von Kohlenwasserstoffen gemäß den Oberbegriffen der jeweiligen unabhängigen Patentansprüche.

### Stand der Technik

Kurzkettige Olefine wie Ethylen und Propylen können durch Dampfspalten (engl. Steam Cracking) von Kohlenwasserstoffen hergestellt werden. Verfahren und Vorrichtungen zum Dampfspalten von Kohlenwasserstoffen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben.

Ein alternativer Weg zu kurzkettigen Olefinen sind die sogenannten Oxygenat-zu-Olefin-Verfahren (engl. Oxygenates to Olefins, OTO). In Oxygenat-zu-Olefin-Verfahren werden Oxygenate wie Methanol oder Dimethylether in eine Reaktionszone eines Reaktors eingebracht, in der ein zur Umsetzung der Oxygenate geeigneter Katalysator bereitgestellt ist. Die Oxygenate werden beispielsweise zu Ethylen und Propylen umgesetzt. Die verwendeten Katalysatoren und Reaktionsbedingungen in Oxygenat-zu-Olefin-Verfahren sind dem Fachmann grundsätzlich bekannt.

Oxygenat-zu-Olefin-Verfahren können mit unterschiedlichen Katalysatoren durchgeführt werden. Beispielsweise können Zeolithe wie ZSM-5 oder SAPO-34 oder funktionell vergleichbare Materialien zum Einsatz kommen. Wenn ZSM-5 oder ein vergleichbares Material zum Einsatz kommt, bilden sich vergleichsweise große Mengen an längerkettigen (C3plus-)Kohlenwasserstoffen (zur Bezeichnung siehe unten) und eher geringe Mengen an kürzerkettigen (C2minus-)Kohlenwasserstoffen. Bei der Verwendung von SAPO-34 oder vergleichbaren Materialien werden im Gegensatz dazu eher kürzerkettige (C2minus-)Kohlenwasserstoffe gebildet.

Auch integrierte Verfahren und Anlagen ("Verbundanlagen") zur Herstellung von Kohlenwasserstoffen, die Dampfspaltverfahren und Oxygenat-zu-Olefin-Verfahren bzw. entsprechende Spaltöfen und Reaktoren umfassen, sind bekannt und beispielsweise in der WO 2011/057975 A2 oder der US 2013/0172627 A1 beschrieben.

Derartige integrierte Verfahren sind beispielsweise deshalb vorteilhaft, weil in den Oxygenat-zu-Olefin-Verfahren typischerweise nicht ausschließlich die gewünschten kurzkettigen Olefine gebildet werden. Ein wesentlicher Teil der Oxygenate wird in Paraffine und C4plus-Olefine umgewandelt. Gleichzeitig wird beim Dampfspalten nicht der gesamte Ofeneinsatz in kurzkettige Olefine gespalten. Im Spaltgas entsprechender Spaltöfen können insbesondere noch nicht umgesetzte Paraffine enthalten sein. Ferner finden sich hier typischerweise C4plus-Olefine, darunter Diolefine wie Butadien. Die erhaltenen Verbindungen richten sich in beiden Fällen nach den verwendeten Einsätzen und Reaktionsbedingungen.

In den in der WO 2011/057975 A2 und der US 2013/0172627 A1 vorgeschlagenen Verfahren werden das Spaltgas eines Spaltofens und der Abstrom eines Oxygenat-zu-Olefin-Reaktors in einer gemeinsamen Trenneinheit kombiniert und fraktioniert. Eine hier gewonnene C4-Fraktion kann, beispielsweise nach Hydrierung oder Abtrennung von Butadien, erneut einem Dampfspalt- und/oder einem Oxygenat-zu-Olefin-Verfahren unterworfen werden. Die C4-Fraktion kann in überwiegend olefinische und überwiegend paraffinische Teilfraktionen getrennt werden.

Die vorliegende Erfindung ist nicht auf Oxygenat-zu-Olefin-Verfahren beschränkt, sondern kann grundsätzlich mit beliebigen katalytischen Verfahren eingesetzt werden, insbesondere mit katalytischen Verfahren, bei denen die zuvor erläuterten Zeolithe als Katalysatoren zum Einsatz kommen. Als Einsatz in entsprechenden katalytischen Verfahren können neben Methanol und/oder Dimethylether auch andere Oxygenate verwendet werden, beispielsweise andere Alkohole und/oder Ether.

Ebenso können olefinische Komponenten, wie beispielsweise eine Mischung aus unterschiedlichen ungesättigten C4-Kohlenwasserstoffen, in entsprechenden katalytischen Verfahren eingesetzt werden. Man spricht in diesem Fall von einem Olefinspaltverfahren (engl. Olefins Cracking Process, OCP). Unterschiedliche Einsätze können im Rahmen der vorliegenden Erfindung in denselben Reaktor oder in unterschiedliche Reaktoren geführt werden. Beispielsweise kann in einem Reaktor ein Oxygenat-zu-Olefin-Verfahren und einem anderen Reaktor ein Olefinspaltverfahren durchgeführt werden. Beide Prozesse, aber auch ggf. ein kombinierter Prozess, haben jedoch zum Ziel, aus einem oder mehreren Einsätzen ein Produkt zu erzeugen, das reich an Propylen und ggf. Ethylen ist.

Die erläuterten katalytischen Verfahren, die sich insbesondere dadurch auszeichnen, dass dabei die erwähnten Zeolithe als Katalysatoren zum Einsatz kommen und ferner ein oder mehrere, Oxygenate und/oder Olefine enthaltende Katalyseeinsatzströme verwendet werden, werden damit in einer Katalyseeinheit durchgeführt, die einen oder mehrere entsprechender Reaktoren aufweisen kann.

Wie bereits erwähnt, ist Ziel entsprechender katalytischer Verfahren, Produkte zu erzeugen, die reich an Propylen und ggf. Ethylen sind. Typischerweise werden in entsprechenden Verfahren aber auch signifikante Mengen von Kohlenwasserstoffen mit vier und mehr Kohlenstoffatomen erzeugt. Aus dem Stand der Technik ist daher bekannt, entsprechende Kohlenwasserstoffe mit vier oder mehr Kohlenstoffatomen zur Katalyse zu rezyklieren. Auch eine Entnahme entsprechender Kohlenwasserstoffe als Produkt(e) ist bekannt. Ferner wurde bereits vorgeschlagen, die Kohlenwasserstoffe mit vier und mehr Kohlenwasserstoffatomen oder Teile bzw. Fraktionen hiervon weiteren Reaktionsprozessen zuzuführen.

Sämtliche der bekannten Verfahren weisen jedoch Nachteile auf. Insbesondere ist die Effizienz der Verwertung entsprechender Kohlenwasserstoffe in solchen Verfahren häufig nicht zufriedenstellend.

Es besteht daher der Bedarf nach Verbesserungen bei entsprechenden Verfahren und Anlagen zur Herstellung von Kohlenwasserstoffen unter Verwendung der erläuterten katalytischen Verfahren.

### Offenbarung der Erfindung

Diese Aufgabe wird durch ein Verfahren und eine Anlage zur Herstellung von Kohlenwasserstoffen mit den Merkmalen der unabhängigen Patentansprüche gelöst. Bevorzugte Ausgestaltungen sind Gegenstand der abhängigen Patentansprüche sowie der nachfolgenden Beschreibung.

Vor der detaillierten Erläuterung der Merkmale und Vorteile der vorliegenden Erfindung werden deren Grundlagen und die verwendeten Begriffe erläutert.

Flüssige und gasförmige Ströme können im hier verwendeten Sprachgebrauch reich oder arm an einer oder mehreren Komponenten sein, wobei "reich" für einen Gehalt von wenigstens 75%, 90%, 95%, 99%, 99,5%, 99,9% oder 99,99% und "arm" für einen Gehalt von höchstens 25%, 10%, 5%, 1%, 0,1% oder 0,01% auf molarer, Gewichts- oder Volumenbasis stehen kann. Der Begriff "überwiegend" kann der soeben getroffenen Definition von "reich" entsprechen, bezeichnet jedoch insbesondere einen Gehalt von mehr als 90%. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ein flüssiger oder gasförmiger Strom ist von einem anderen flüssigen oder gasförmigen Strom (auch als Ausgangsstrom bezeichnet) "abgeleitet" oder aus diesem "gebildet", wenn er zumindest einige in dem Ausgangsstrom enthaltene oder aus diesem erhaltene Komponenten aufweist. Ein in diesem Sinne abgeleiteter oder gebildeter Strom kann aus dem Ausgangsstrom insbesondere durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend oder ausschließlich Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem und zwei Kohlenstoffatomen.

Unter Oxygenaten werden typischerweise Ether und Alkohole verstanden. Neben Methyl-*tert*-butylether (MTBE, engl. methyl tertiary butyl ether) kommen beispielsweise *tert*-Amylmethylether (TAME, tertiary amyl methyl ether), *tert*-Amylethylether (TAEE, tertiary amyl ethyl ether), Ethyl-*tert*-butylether (ETBE, ethyl tertiary butyl ether) und Diisopropylether (DIPE, diisopropyl ether) zum Einsatz. Als Alkohole werden beispielsweise Methanol, Ethanol und *tert*-Butanol (TBA, tertiary butyl alcohol) verwendet. Zu den Oxygenaten zählt insbesondere auch Dimethylether (DME, dimethyl ether). Die Erfindung eignet sich auch zur Verwendung mit anderen Oxygenaten.

Gemäß einer gängigen Definition, die auch hier Anwendung findet, handelt es sich bei Oxygenaten um Verbindungen, die wenigstens eine kovalent an ein Sauerstoffatom gebundene Alkylgruppe aufweisen. Die wenigstens eine Alkylgruppe kann bis zu fünf, bis zu vier oder bis zu drei Kohlenstoffatome aufweisen. Insbesondere weisen die Oxygenate, die im Rahmen der vorliegenden Erfindung von Interesse sind, Alkylgruppen mit einem oder zwei Kohlenstoffatomen auf, insbesondere handelt es sich um Methylgruppen. Insbesondere handelt es sich um einwertige Alkohole und Dialkylether wie Methanol und Dimethylether oder entsprechende Mischungen.

Dampfspaltverfahren werden im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden auch jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine im Rahmen der vorliegenden Erfindung eingesetzte Dampfspalteinheit kann einen oder mehrere derartiger Spaltöfen aufweisen.

Entsprechendes gilt auch, wie bereits angesprochen, für die im Rahmen der vorliegenden Erfindung verwendete Katalyseeinheit, in der unterschiedliche Reaktoren mit gleichen oder unterschiedlichen Katalysatoren ausgestattet, mit gleichen oder unterschiedlichen Einsatzströmen beschickt und bei gleichen oder unterschiedlichen Reaktionsbedingungen betrieben werden können.

Mit dem Begriff "Dampfspalteinsatzströme" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und erneut als Dampfspalteinsatzströme verwendet werden. Als Dampfspalteinsatzströme eignen sich eine Vielzahl von Kohlenwasserstoffen und Kohlenwasserstoffgemischen von Ethan bis Gasöl bis zu einem Siedepunkt von typischerweise 600 °C.

Ein Dampfspalteinsatzstrom kann ausschließlich sogenannten "Frischeinsatz" umfassen, also einen Einsatz, der anlagenextern bereitgestellt und beispielsweise aus einer oder mehreren Erdölfraktionen, Erdgas und/oder Erdgaskondensaten gewonnen wird. Ein Dampfspalteinsatzstrom kann aber auch zusätzlich oder ausschließlich einen oder mehrere sogenannte "Recycleströme" umfassen, also Ströme, die in der Anlage selbst erzeugt und in einen entsprechenden Spaltofen zurückgeführt werden. Ein Dampfspalteinsatzstrom kann also auch aus einem Gemisch eines oder mehrerer Frischeinsätze mit einem oder mehreren Recycleströmen bestehen.

Der Dampfspalteinsatzstrom wird im jeweiligen Spaltofen zumindest teilweise umgesetzt und verlässt den Spaltofen als sogenanntes "Rohgas", das Nachbehandlungsschritten unterworfen werden kann. Diese Nachbehandlungsschritte umfassen zunächst eine Aufbereitung des Rohgases, beispielsweise durch Quenchen, Kühlen und Trocknen, wodurch ein "Spaltgas" erhalten wird. Bisweilen wird auch bereits das Rohgas als Spaltgas bezeichnet. Im vorliegenden Fall wird hierfür der Begriff "Dampfspaltproduktstrom" verwendet.

Wiederum gilt Entsprechendes auch für den oder die einer oder mehreren Katalyseeinheiten zugeführten Einsatzströme, die hier als "Katalyseeinsatzströme" bezeichnet werden. Der oder die Katalyseeinsatzströme werden in der Katalyseeinheit in einem oder mehreren Reaktoren zu einem oder mehreren Produktströmen, die hier als "Katalyseproduktströme" bezeichnet werden, umgesetzt.

In neueren Verfahren und Vorrichtungen zum Dampfspalten kommen zunehmend milde Spaltbedingungen zum Einsatz, weil sich bei diesen insbesondere sogenannte Wertprodukte, beispielsweise Propylen, in größerer Menge gewinnen lassen. Grundsätzlich sind Verfahren von Vorteil, bei denen die Spaltbedingungen an die Zusammensetzung des oder der Dampfspalteinsatzströme angepasst werden. Bei milden Spaltbedingungen reduziert sich jedoch auch die Umsetzung in dem oder den Spaltöfen, so dass sich nicht umgesetzte Verbindungen in vergleichsweise großer Menge in dem oder den Dampfspaltproduktströmen wiederfinden und auf diese Weise zu einer "Verdünnung" der zu gewinnenden Wertprodukte führen.

Die erwähnten "Spaltbedingungen" in einem Spaltofen umfassen unter anderem den Partialdruck des Ofeneinsatzes, der durch die Zugabe unterschiedlicher Dampfmengen und den im Spaltofen eingestellten Druck beeinflusst werden kann, die Verweildauer im Spaltofen sowie die in diesem verwendeten Temperaturen und Temperaturprofile. Auch die Ofengeometrie und Ofengestaltung spielt eine Rolle.

Da die genannten Werte einander zumindest teilweise wechselseitig beeinflussen, hat sich zur Charakterisierung der Spaltbedingungen der Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden. Für gasförmige Ofeneinsätze kann dagegen die Umsetzung bzw. Konversion einer jeweils betrachteten Komponente des Ofeneinsatzes als Maß der Spaltschärfe angegeben werden. Insbesondere für Kohlenwasserstoffe mit vier Kohlenstoffatomen ist eine Beschreibung der Spaltschärfe über die Umsetzung von Schlüsselkomponenten wie *n-*Butan und Isobutan gut geeignet. Zum fachmännischen Verständnis des Begriffs der Spaltschärfe (engl. Severity) sei beispielsweise auf den erwähnten Artikel "Ethylene" in Ullmann's Encyclopedia of Industrial Chemistry verwiesen.

### Vorteile der Erfindung

Die vorliegende Erfindung kombiniert die eingangs erläuterten Maßnahmen zur Nutzung von Kohlenwasserstoffen wie mit vier oder mehr Kohlenstoffatomen aus einem entsprechenden Katalyseverfahren in optimierter Weise, so dass insgesamt eine effiziente Verwertung mit maximaler Wertschöpfung und geringen internen Recycleströmen erfolgt.

Zu diesem Zweck geht die vorliegende Erfindung von einem Verfahren zur Herstellung von Kohlenwasserstoffen aus, das umfasst, in einer Katalyseeinheit unter Verwendung eines oder mehrerer, Oxygenate und/oder Olefine enthaltender Katalyseeinsatzströme einen n-Butan, Isobutan, 1-Buten, 2-Buten, Isobuten und Kohlenwasserstoffe mit mehr als vier und/oder weniger als 3 Kohlenstoffatomen enthaltenden Katalyseproduktstrom zu erzeugen. Die Katalyseeinheit umfasst dabei, wie zuvor erläutert, einen oder mehrere Reaktoren, die mit einem oder mehreren Einsatzströmen, die hier als Katalyseeinsatzströme bezeichnet werden, beschickt werden. Wie erläutert, eignet sich die vorliegende Erfindung bei Oxygenat-zu-Olefin-Verfahren und/oder den sogenannten Olefinspaltverfahren. Der oder die in einer entsprechenden Katalyseeinheit verwendeten Reaktoren weisen vorzugsweise Zeolithe als Katalysatoren auf. Wie erläutert, können diese Katalysatoren insbesondere vom SAPO- oder ZSM-Typ sein. Die im Rahmen der vorliegenden Erfindung eingesetzte Katalyseeinheit ist damit für ein entsprechendes Katalyseverfahren eingerichtet.

Ferner sieht das Verfahren vor, in einer Dampfspalteinheit unter Verwendung eines oder mehrerer Dampfspalteinsatzströme einen Dampfspaltproduktstrom zu erzeugen. Das im Rahmen der vorliegenden Erfindung eingesetzte Dampfspaltverfahren kann dabei unter Verwendung gleicher oder unterschiedlicher Dampfspaltbedingungen in einem oder mehreren Spaltöfen erfolgen, wie grundsätzlich bekannt. Zu Details wird auf die obigen Erläuterungen verwiesen. Insbesondere können die beim Dampfspalten eingesetzten Dampfspalteinsatzströme mild gespalten werden, um die Ausbeute an Wertprodukten zu erhöhen. Schärfere Spaltbedingungen können insbesondere eingesetzt werden, um einen möglichst hohen Umsatz zu erzielen.

Erfindungsgemäß ist vorgesehen, dass aus dem Katalyseproduktstrom oder einem Teil hiervon zumindest der überwiegende Teil der Kohlenwasserstoffe mit mehr als vier und/oder weniger als drei Kohlenstoffatomen und des Isobutens entfernt wird und ein oder mehrere jeweils verbleibende Ströme als der oder die Dampfspalteinsatzströme verwendet werden. Mit anderen Worten sieht die vorliegende Erfindung vor, aus dem in einer entsprechenden Katalyseeinheit erhaltenen Katalyseproduktstrom zunächst einen C4-Schnitt zu erzeugen, nämlich durch die erwähnte Entfernung des überwiegenden Anteils der Kohlenwasserstoffe mit mehr als vier und/oder weniger als drei Kohlenstoffatomen.

Ist hier und im Folgenden von einer Entfernung "des überwiegenden Teils" entsprechender Kohlenwasserstoffe die Rede, kann hiervon umfasst sein, dass, wie einleitend erläutert, mindestens 75% oder mehr solcher Kohlenwasserstoffe entfernt werden. Vorzugsweise werden entsprechende Kohlenwasserstoffe im Wesentlichen vollständig entfernt, also insbesondere zu mindestens 90 oder 95%, gegebenenfalls auch zu wenigstens 99% oder mehr.

Ferner enthält ein derartiger C4-Schnitt, der durch die Abtrennung des überwiegenden Teils der Kohlenwasserstoffe mit mehr als vier und/oder weniger als drei Kohlenstoffatomen erzeugt wird, vergleichsweise wenig Diene, ist also "arm" an entsprechenden Dienen im oben erläuterten Sinn. Dies ergibt sich aus dem zuvor durchgeführten Katalyseverfahren, bei dem entsprechend wenige Diene, beispielsweise Butadien, gebildet werden.

In einem oder mehreren weiteren Schritten wird im Rahmen der vorliegenden Erfindung das Isobuten aus dem erzeugten C4-Schnitt möglichst selektiv entfernt. Isobuten sollte deshalb vor der Durchführung eines entsprechenden Dampfspaltverfahrens möglichst vollständig entfernt werden, da es sich in einem entsprechenden Dampfspaltverfahren vergleichsweise schlecht umsetzen lässt und sich damit in vergleichsweise hoher Konzentration in dem oder den Dampfpaltproduktströmen wiederfindet.

Der verbleibende Rest, aus dem jedoch noch weitere Komponenten abgetrennt werden können, wird in Form eines oder mehrerer Ströme der verwendeten Dampfspalteinheit zugeführt, wo er bei gleichen oder unterschiedlichen Spaltbedingungen in einem oder mehreren Spaltöfen gespalten wird. Wie unten näher erläutert, kann aus dem Dampfspaltproduktstrom, der in einer entsprechenden Dampfspalteinheit erhalten wird, erneut ein entsprechender C4-Schnitt gewonnen werden, aus dem insbesondere Butadien als Produkt abgetrennt werden kann. Ein solcher, an Butadien abgereicherter C4-Schnitt kann dann an geeigneter Stelle recycelt werden, beispielsweise mit dem in der Katalyseeinheit erhaltenen Katalyseproduktstrom oder einem hieraus erzeugten C4-Schnitt vereinigt werden.

Das im Rahmen der vorliegenden Erfindung vorgeschlagene Verfahren ist besonders effizient, da hier das für die Dampfspaltung ungeeignete Isobuten entfernt wird, jedoch alle anderen Komponenten weitgehend als Dampfspalteinsatzstrom bzw. Dampfspaltströme der Dampfspaltung zugeführt werden können. Auf diese Weise ist es auch möglich, die Produktmenge von Butadien, die in der Dampfspaltung gebildet wird, zu maximieren, da die Spaltbedingungen bei Abwesenheit von Isobuten weitgehend in Richtung einer maximalen Butadienproduktion angepasst werden können. Das entfernte Isobuten, bzw. eine entsprechende Fraktion, die Isobuten oder ein hieraus erzeugtes Produkt enthält, kann entweder als Produkt abgeführt oder in die Katalyseeinheit zurückgeführt werden.

Ein wesentlicher Unterschied im Rahmen der vorliegenden Erfindung gegenüber einem bekannten Verfahren, wie es beispielsweise in der US2013/0172627 A1 offenbart ist, besteht darin, dass paraffinische und olefinische Komponenten, außer Isobuten, zum Dampfspalten geführt werden können und nicht lediglich eine Trennung in C4-Olefine und C4-Paraffine erfolgt.

Nachfolgend werden bereits teilweise erläuterte Ausführungsformen der Erfindung, wie sie in den abhängigen Patentansprüchen wiedergegeben sind, nochmals zusammengefasst erläutert.

Insbesondere sieht das erfindungsgemäße Verfahren vor, dass zunächst der überwiegende Teil der Kohlenwasserstoffe mit mehr als vier und/oder weniger als drei Kohlenstoffatomen unter Verbleib eines C4-Teilstroms abgetrennt wird und anschließend der überwiegende Teil des Isobutens aus dem C4-Teilstrom entfernt wird. Vorteile entsprechender Maßnahmen wurden bereits zuvor erläutert.

Gemäß einer besonders bevorzugten Ausführungsform der Erfindung ist vorgesehen, dass das Entfernen des überwiegenden Teils des Isobutens die zumindest teilweise Umsetzung des Isobutens und die Abtrennung zumindest eines Teils des dabei gebildeten Umsetzungsprodukts umfasst. Entsprechende Maßnahmen erleichtern die Entfernung von Isobuten beträchtlich, so dass auf aufwändige Abtrenneinrichtungen verzichtet werden kann. Insbesondere eignet sich für die zumindest teilweise Umsetzung des Isobutens ein katalytisches Verfahren, bei dem das Isobuten mit Methanol und/oder Ethanol zu Methyl- oder Ethyl-*tert*-Butylether umgesetzt wird. Entsprechende Verfahren sind grundsätzlich aus dem Stand der Technik bekannt. Gewonnener Ethyl- oder Methyl-*tert*-Butylether kann aus einem entsprechenden C4-Teilstrom einfach abgetrennt und als Produkt bereitgestellt werden. Methyl-*tert-*Butylether eignet sich insbesondere als Antiklopfmittel bzw. als Lösungsmittel in der chemischen Industrie. Entsprechende Produkte können jedoch auch in die Katalyseeinheit zurückgeführt und dort weiter umgesetzt werden.

Im Rahmen eines erfindungsgemäßen Verfahrens kann zumindest ein Teil eines nach dem Entfernen des überwiegenden Teils des Isobutens verbleibenden Stroms als der oder einer der Dampfspalteinsatzströme verwendet werden, wenn keine weitere Abtrennung von Komponenten gewünscht ist. Weil ein entsprechender Strom nurmehr wenig oder im Wesentlichen kein Isobuten enthält, kann das Dampfspaltverfahren flexibel an die jeweils zu gewinnenden Produkte angepasst werden.

Alternativ zur zumindest teilweisen Umsetzung des Isobutens und der Abtrennung zumindest eines Teils des dabei gebildeten Umsetzungsprodukts kann ein erfindungsgemäßes Verfahren auch vorsehen, den überwiegenden Teil des Isobutens destillativ abzutrennen. Eine destillative Abtrennung hat den Vorteil, dass Isobuten direkt, d. h. nicht als entsprechendes Umsetzungsprodukt, gewonnen werden kann.

Um eine entsprechende destillative Abtrennung zu erleichtern, ist es besonders vorteilhaft, vor der destillativen Abtrennung des überwiegenden Teils des Isobutens den überwiegenden Teil des in dem C4-Teilstrom enthaltenen 1-Butens zu 2-Buten zu isomerisieren. Der Siedepunkt von Isobuten bei Atmosphärendruck liegt bei -6,9 °C, jener von 1-Buten bei -6,47 °C. Eine destillative Trennung ist damit praktisch nicht möglich. Der Siedepunkt von 2-Buten liegt hingegen deutlich oberhalb hiervon, nämlich bei 3,7 °C für cis-2-Buten und bei 0,9°C für *trans*-2-Buten.

Es kann auch vorteilhaft sein, aus zumindest einem Teil eines nach dem Entfernen des überwiegenden Teils des Isobutens verbleibenden Stroms einen überwiegend Butan und 2-Buten enthaltenden Strom zu bilden und diesen als den Dampfspalteinsatzstrom oder einen der Dampfspalteinsatzströme zu verwenden. Mit anderen Worten wird hier nach dem Entfernen des überwiegenden Teils des Isobutens auch eine Entfernung anderer Komponenten als der genannten durchgeführt. Die genannten Komponenten, nämlich n-Butan und 2-Buten, eignen sich in besonderer Weise als Dampfspalteinsätze.

Zusätzlich kann es vorteilhaft sein, aus den bei der Bildung eines entsprechenden, überwiegend n-Butan und 2-Butan enthaltenden Stroms abgetrennten Komponenten einen Strom zu bilden, der überwiegend Isobutan enthält und diesen als den Dampfspalteinsatzstrom oder einen der Dampfspalteinsatzströme zu verwenden. Beispielsweise kann im Rahmen der vorliegenden Erfindung vorgesehen sein, aus einem Isobuten, Isobutan und gegebenenfalls 1-Buten sowie n-Butan und 2-Buten enthaltenden Strom einen überwiegend n-Butan und 2-Buten enthaltenden Strom zu bilden und diesen der Dampfspalteinheit zuzuführen. Ein verbleibender, Isobuten, Isobutan und ggf. 1-Buten enthaltender Strom kann einem Destillationsverfahren unterworfen werden, in dem das Isobutan abgetrennt wird. Dieses kann, wie erwähnt, in der Dampfspalteinheit behandelt werden.

Wie erwähnt, enthält ein entsprechender Dampfspaltproduktstrom Kohlenwasserstoffe mit vier Kohlenstoffatomen, darunter Butadien, sowie Kohlenwasserstoffe mit mehr als vier und/oder weniger als drei Kohlenstoffatomen. Eine Zusammensetzung eines entsprechenden Stroms kann, wie erwähnt, durch Einstellen der verwendeten Spaltbedingungen optimiert werden.

Vorteilhafterweise wird der überwiegende Teil des Butadiens und der Kohlenwasserstoffe mit mehr als vier und/oder mit weniger als drei Kohlenstoffatomen aus dem Dampfspaltproduktstrom unter Erhalt eines butadienarmen Reststroms abgetrennt, welcher überwiegend Kohlenwasserstoffe mit vier Kohlenstoffatomen enthält. Dies ermöglicht die weitgehende Rückgewinnung des Butadiens und die Rückführung entsprechender anderer Verbindungen. Vorteilhafterweise wird daher zumindest ein Teil des butadienarmen Reststroms mit zumindest einem Teil des zuvor erläuterten C4-Teilstroms vereinigt.

Eine erfindungsgemäße Anlage ist zur Herstellung von Kohlenwasserstoffen eingerichtet. Sie umfasst zumindest eine Katalyseeinheit, die dazu eingerichtet ist, unter Verwendung eines oder mehrerer, Oxygenate und/oder Olefine enthaltender Katalyseeinsatzströme einen n-Butan, Isobutan, 1-Buten, 2-Buten, Isobuten und Kohlenwasserstoffe mit mehr als 4 und/oder weniger als drei Kohlenstoffatomen enthaltenden Katalyseproduktstrom zu erzeugen.

Ferner umfasst eine derartige Anlage eine Dampfspalteinheit, die dazu eingerichtet ist, unter Verwendung eines oder mehrerer Dampfspalteinsatzströme einen Dampfspaltproduktstrom zu erzeugen. Erfindungsgemäß sind in einer entsprechenden Anlage Mittel vorgesehen, die dazu eingerichtet sind, aus dem Katalyseproduktstrom oder einem Teil hiervon zumindest den überwiegenden Anteil der Kohlenwasserstoffe mit mehr als vier und/oder weniger als drei Kohlenstoffatomen und des Isobutens zu entfernen und einen oder mehrere jeweils verbleibende Ströme als den oder die Dampfspalteinsatzströme zu verwenden.

Zu Merkmalen und Vorteilen einer entsprechenden Anlage sei auf die obigen Erläuterungen bezüglich des erfindungsgemäßen Verfahrens ausdrücklich verwiesen. Insbesondere ist eine entsprechende Anlage durch geeignete Mittel dazu eingerichtet, sämtliche Schritte eines entsprechenden Verfahrens durchzuführen.

Die Erfindung und bevorzugte Ausführungsformen der Erfindung werden nachfolgend unter Bezugnahme auf die beigefügten Zeichnungen näher erläutert, die bevorzugte Ausführungsformen der Erfindung zeigen.

### Kurze Beschreibung der Zeichnungen

Figur 1 zeigt eine Anlage gemäß einer Ausführungsform der Erfindung in schematischer Darstellung.
Figur 2 zeigt eine Anlage gemäß einer Ausführungsform der Erfindung in schematischer Darstellung.
Figur 3 zeigt eine Anlage gemäß einer Ausführungsform der Erfindung in schematischer Darstellung.

### Ausführungsformen der Erfindung

In den Figuren sind einander entsprechende Elemente mit identischen Bezugszeichen angegeben und werden der Übersichtlichkeit halber nicht wiederholt erläutert. Die in den jeweiligen Figuren gezeigten Ströme sind dann mit identischen Bezugszeichen versehen, wenn sie im Wesentlichen gleiche oder vergleichbare Zusammensetzung aufweisen und, ungeachtet möglicherweise unterschiedlicher Volumenströme. In allen

Figuren ist eine Katalyseeinheit mit 1 und eine Dampfspalteinheit mit 2 bezeichnet.

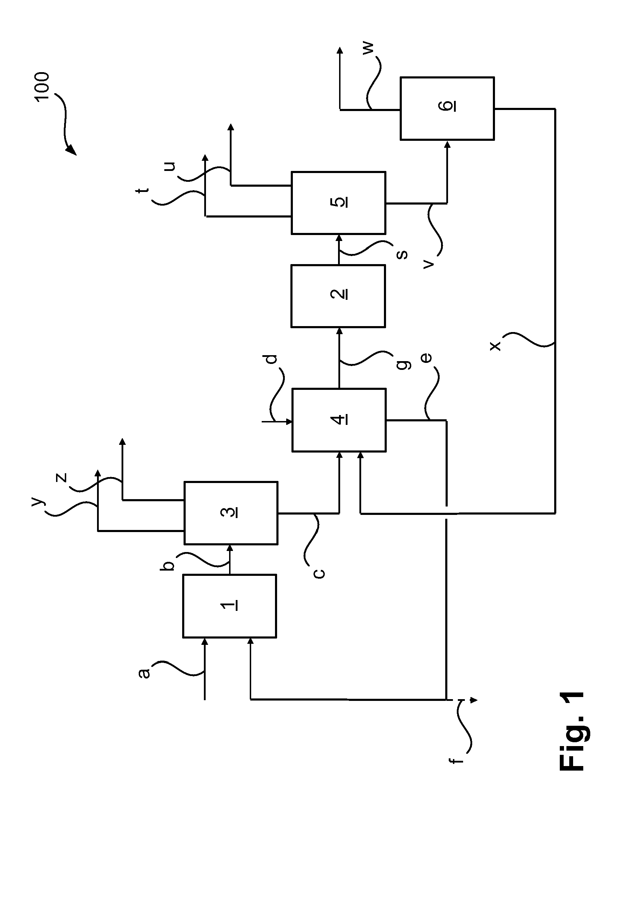

In Figur 1 ist eine Anlage gemäß einer Ausführungsform der Erfindung vereinfacht schematisch dargestellt und insgesamt mit 100 bezeichnet.

Der Katalyseeinheit 1 werden hier ein oder mehrere, Oxygenate und/oder Olefine enthaltende Katalyseeinsatzströme, hier mit a bezeichnet, zugeführt. Wie erwähnt, kann die Katalyseeinheit 1 einen oder mehrere Reaktoren umfassen, die mit einem Zeolithkatalysator betrieben werden. Die Katalyseeinheit kann zusätzlich mit weiteren Strömen, hier dem Strom e, beschickt werden, wie unten erläutert.

In der Katalyseeinheit 1 wird im dargestellten Beispiel ein Katalyseproduktstrom b erzeugt. Dieser wird einer Trenneinheit 3 zugeführt, in der aus dem Katalyseproduktstrom b ein an Kohlenwasserstoffen mit mehr als vier und/oder weniger als drei Kohlenstoffatomen abgereicherter bzw. an Kohlenwasserstoffen mit vier Kohlenstoffatomen reicher Strom c, also ein C4-Strom, erhalten wird. Die dabei abgetrennten Ströme, hier mit y und z bezeichnet, können beispielsweise Kohlenwasserstoffe mit fünf und mehr oder Kohlenwasserstoffe mit drei und weniger Kohlenstoffatomen oder entsprechende andere Fraktionen umfassen. Solche Ströme können ebenfalls in einer entsprechenden Anlage bearbeitet und/oder als Produkte gewonnen werden.

Der C4-Strom c wird im dargestellten Beispiel einer Reaktionseinheit 4 zugeführt, in der in dem C4-Strom c enthaltenes Isobuten beispielsweise mit Methanol, das in Form eines Stroms d zugeführt wird, zu Methyl-*tert*-Butylether umgesetzt wird. Methyl-*tert-*Butylether oder ein anderes Umsetzungsprodukt mit einer anderen, in Form des Stroms d zugeführten Verbindung kann als Strom e abgezogen werden. Wie mit einem gestrichelten Pfeil veranschaulicht, kann optional zumindest ein Teil entsprechender Produkte als Strom f aus der Anlage ausgeführt werden. Der verbleibende Anteil des Stroms e oder der gesamte Strom e kann erneut in die Katalyseeinheit 1 geführt und damit als Recyclestrom verwendet werden.

Ein auf diese Weise von Isobuten befreiter C4-Strom, nun mit g bezeichnet, wird der Dampfspalteinheit 2 zugeführt und dort in einem oder mehreren Spaltöfen, gegebenenfalls auch zusammen mit weiteren Strömen, die in gleiche oder unterschiedliche Spaltöfen geführt werden, bearbeitet. Es wird ein Dampfspaltproduktstrom s erhalten, der, wie erläutert, Kohlenwasserstoffe mit vier Kohlenstoffatomen, darunter Butadien, sowie Kohlenwasserstoffe mit mehr als vier und/oder weniger als drei Kohlenstoffatomen enthält. Dieser Strom, hier mit s bezeichnet, wird einer weiteren Trenneinheit 5 zugeführt, in der zunächst unter Abtrennung von Kohlenwasserstoffen mit mehr als vier und/oder weniger als drei Kohlenstoffatomen, wie hier mit den Strömen t und u veranschaulicht, ein Strom v erhalten wird, der überwiegend Kohlenwasserstoffe mit vier Kohlenstoffatomen, darunter Butadien, enthält. Der Strom v wird im dargestellten Beispiel in eine Butadienabtrenneinheit 6 geführt, wo das enthaltende Butadien zu einem überwiegenden Anteil abgetrennt und als Strom w aus der Anlage ausgeführt wird. Ein verbleibender, butadienarmer Reststrom, hier mit x bezeichnet, kann mit dem zuvor erläuterten C4-Teilstrom c vereinigt und in die Reaktionseinheit 4 geführt werden.

Je nach Zielsetzung kann in der Dampfspalteinheit 2 eine scharfe Spaltung (zur Ethylenmaximierung) oder milde Spaltung (zur Propylenmaximierung) erfolgen. Unabhängig von der Spaltschärfe wird jedoch tendenziell eine höhere Menge Butadien erzeugt, da der verwendete Einsatz in Form des Stroms g noch ungesättigte Komponenten, nämlich 1-Buten und 2-Buten enthält. Ist eine erhöhte Butadienmenge nicht erwünscht, können der oder die Dampfspalteinsatzströme zuvor hydriert werden. Auf diese Weise kann eine Propylen- bzw. Ethylenausbeute weiter gesteigert werden. Eine (Teil-)Hydrierung und/oder Hydroisomerisierung, die ebenfalls vorgesehen sein kann, kann außerdem dazu eingesetzt werden, ggf. noch vorhandene, mehrfach ungesättigte Komponenten zu hydrieren sowie 1-Buten zu 2-Buten umzusetzen, was wiederum die Butadienerzeugung fördert. Sämtliche Verfahren haben zusammenfassend den Vorteil, dass für die Spaltung in der Dampfspalteinheit 2 das ungeeignete Isobuten entfernt wurde, jedoch sämtliche anderen Komponenten, insbesondere n-Buten, für die Spaltung noch zur Verfügung stehen.

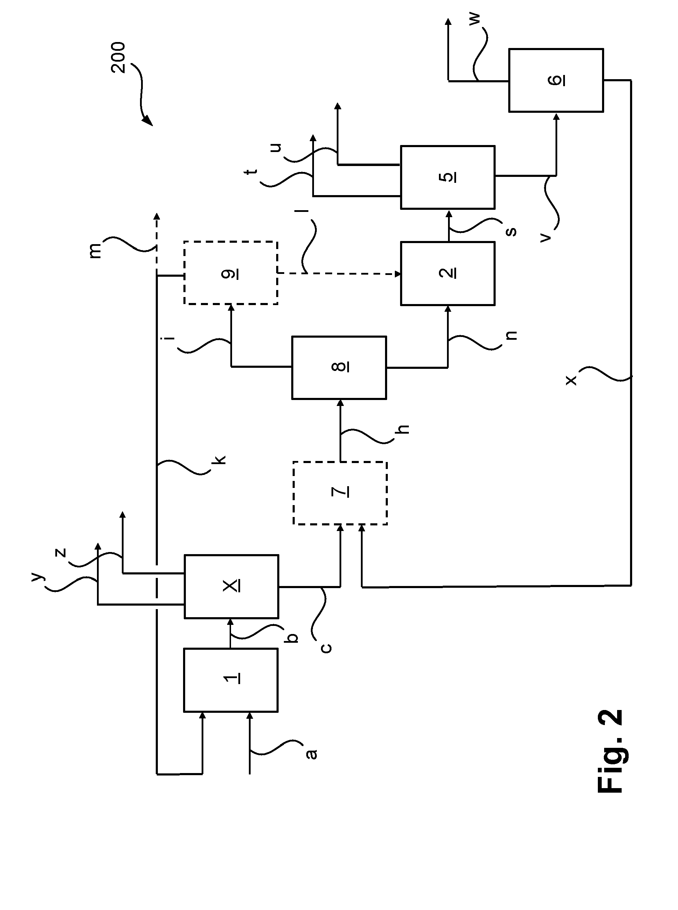

In Figur 2 ist eine Anlage gemäß einer weiteren Ausführungsform der Erfindung schematisch dargestellt und insgesamt mit 200 bezeichnet.

Im Unterschied zu der in der Figur 1 dargestellten Anlage 100 ist hier keine Reaktionseinheit 4 zur Umsetzung des Isobutens vorgesehen, jedoch gegebenenfalls, wie in Form eines gestrichelten Blocks 7 veranschaulicht, eine Isomerisierungseinheit. Die in Figur 1 mit 3 bezeichnete Trenneinheit ist hier mit x bezeichnet, kann aber identisch ausgebildet sein. Die Isomerisierungseinheit 7 ist insbesondere dazu eingerichtet, in dem Strom c (und dem Strom x) enthaltenes 1-Buten zu 2-Buten zu isomerisieren, um eine anschließende Destillation in einer Destillationseinheit 8 zu erleichtern, wie oben erläutert. Die Isomerisierungseinheit 7 ist insbesondere zur Hydroisomerisierung eingerichtet. Durch die Isomerisierung in der Isomerisierungseinheit 7 werden in der Destillationseinheit 8 vorzugsweise ausschließlich verzweigte C4-Komponenten abgetrennt. In der Destillationseinheit 8 wird dabei ein Strom i erhalten, der überwiegend Isobuten und Isobutan enthält, nicht jedoch das ansonsten auch in eine entsprechende Fraktion bzw. den Strom i übergehende 1-Buten, da dieses in der Isomerisierungseinheit 7 isomerisiert wurde. Erfolgt keine entsprechende Isomerisierung, geht 1-Buten ebenfalls in den Strom i über. Ein weiterer in der Destillationseinheit 8 erhaltener Strom, hier mit n bezeichnet, enthält im Wesentlichen Butan und 2-Buten, die als Dampfspalteinsatzstrom verwendet werden und in die Dampfspalteinheit 2 geführt werden.

Der Strom i wird im dargestellten Beispiel optional in einer weiteren Destillationseinheit 9 behandelt, wo aus dem Strom i ein Isobutenstrom k, oder, wenn der Strom i noch 1-Buten enthält, ein Isobuten und 1-Buten enthaltender Strom k erzeugt wird, der in die Katalyseeinheit zurückgeführt werden kann. Ein in der weiteren Destillationseinheit 9 erhaltener Strom I enthält im Wesentlichen Isobutan und wird als Dampfspalteinsatzstrom der Dampfspalteinheit 2 zugeführt.

Stehen in einer entsprechenden Anlage die Isomerisierungseinheit 7 und die weitere Destillationseinheit 9 bereit, können im Wesentlichen sämtliche C4-Kohlenwasserstoffe des Stroms c, außer Isobuten, in die Dampfspalteinheit 2 geführt und dort gewinnbringend bearbeitet werden. Das Isobuten kann, wie erwähnt, in Form des Stroms k in die Katalyseeinheit zurückgeführt und/oder in Form des Stroms m aus einer entsprechenden Anlage ausgeleitet werden. Es besteht ein Potential zur weiteren Maximierung des Butadiengehalts in dem Strom s in der Dampfspalteinheit, da aus 2-Buten tendenziell vermehrt Butadien entsteht. Zu den Strömen s bis x und den Einheiten 5 und 6 sei auf die obigen Erläuterungen zu Figur 1 verwiesen.

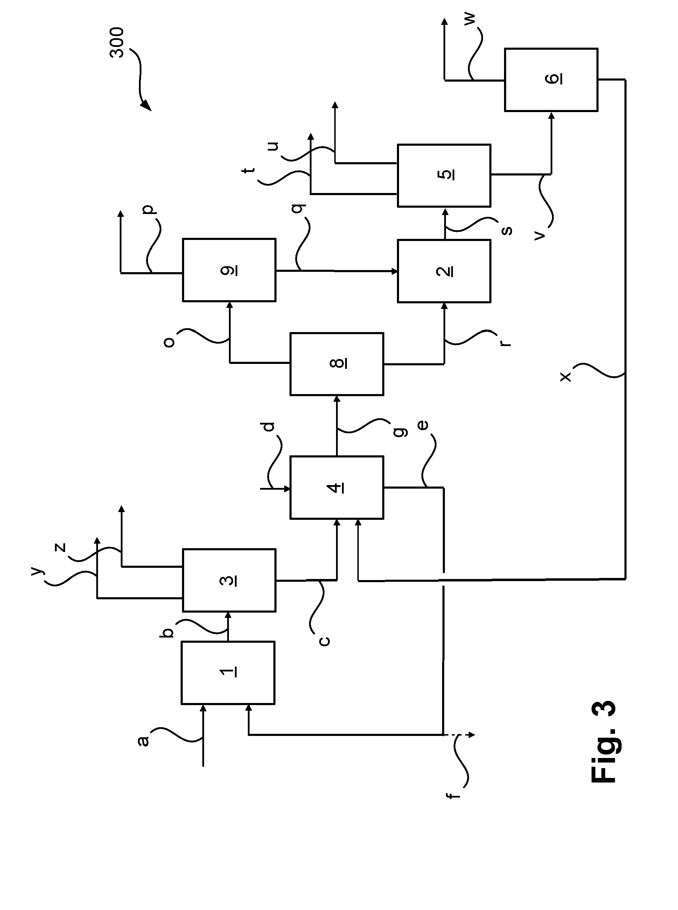

In Figur 3 ist eine Anlage gemäß einer weiteren Ausführungsform der Erfindung schematisch dargestellt und insgesamt mit 300 bezeichnet.

Die Anlage 300 der Figur 3 entspricht bis zur Reaktionseinheit 4 im Wesentlichen der Anlage 100 gemäß Figur 1. Ein auch hier mit g bezeichneter, an Isobuten abgereicherter C4-Kohlenwasserstoffstrom wird hier jedoch in eine Destillationseinheit geführt, die grundsätzlich vergleichbar zu der Destillationseinheit in der Anlage 200 gemäß Figur 2 ausgebildet und daher auch hier mit 8 bezeichnet ist.

In der Destillationseinheit 8 wird aus dem an Isobuten abgereicherten C4-Kohlenwasserstoffstrom g ein Teilstrom o gewonnen, der im Wesentlichen Isobutan und 1-Buten enthält. Dieser wird einer weiteren Destillationseinheit zugeführt, die auch hier mit 9 bezeichnet ist. In der weiteren Destillationseinheit 9 wird aus dem Strom o ein im Wesentlichen 1-Buten enthaltender Strom p und ein im Wesentlichen Isobutan enthaltender Strom q erzeugt. Der Strom q kann, wie ein Strom r, der in der Destillationseinheit 8 erzeugt wird und überwiegend Butan und 2-Buten enthält, als Dampfspalteinsatzstrom der Dampfspalteinheit 2 zugeführt werden. Zu den Strömen s bis x und den Einheiten 5 und 6 sei auf die obigen Erläuterungen zu Figur 1 verwiesen.

Insgesamt können in allen der gezeigten Anlagen 100 bis 300 Kohlenwasserstoffströme mit drei und weniger Kohlenstoffatomen aus den Trenneinheiten 3 bzw. x und 5 gemeinsam weiter verarbeitet werden. Entsprechende, aus diesen Fraktionen abgetrennte Paraffine, insbesondere Ethan und Propan, können in die Dampfspalteinheit 2 zurückgeführt werden. Ähnliches gilt für entsprechende Ströme mit Kohlenwasserstoffen mit fünf und mehr Kohlenstoffatomen. Auch diese können gemeinsam weiter verarbeitet werden. Es kann eine gemeinsame Fraktionierung erfolgen und bestimmte Fraktionen können in die Katalyseeinheit 1 und/oder, gegebenenfalls nach Hydrierung, in die Dampfspalteinheit 2 zurückgeführt werden. Es sei betont, dass in bestimmten Prozessvarianten auch weitere Fraktionen verarbeitet werden können, beispielsweise Fraktionen, die auch C5-, C6- und höhere Kohlenwasserstoffe enthalten.

Weitere Ausführungsformen der vorliegenden Erfindung könnten auch vorsehen, die abgetrennte Isobutenfraktion, die insbesondere noch 1-Buten und Isobutan enthält, voll zu hydrieren und auch einer Dampfspalteinheit 2 zuzuführen, falls jegliche Recycleströme zu der Katalyseeinheit 1 unerwünscht sind. Falls beispielsweise eine Gewinnung von 1-Buten erwünscht ist, ist auch dies durch eine Kombination aus einer entsprechenden Umsetzungseinheit und einer anschließenden Destillation, wie in Figur 3 gezeigt, möglich. Einer Dampfspalteinheit 2 können in allen Fällen auch weitere Einsätze, beispielsweise Naphtha und/oder Ethan bzw. Propan zugeführt werden.

Es sei betont, dass sich ein besonderer Vorteil der vorliegenden Erfindung daraus ergibt, dass die jeweils aus der Katalyseeinheit b enthaltenen Ströme bzw. die aus diesem gebildeten Ströme c geringe Gehalte an Dienen aufweisen.

## Patentansprüche

1. Verfahren zur Herstellung von Kohlenwasserstoffen, das umfasst, in einer Katalyseeinheit (1) unter Verwendung eines oder mehrerer, Oxygenate und/oder Olefine enthaltender Katalyseeinsatzströme (a) einen n-Butan, Isobutan, 1-Buten, 2-Buten, Isobuten und Kohlenwasserstoffe mit mehr als vier und/oder weniger als drei Kohlenstoffatomen enthaltenden Katalyseproduktstrom (b) zu erzeugen, und das ferner umfasst, in einer Dampfspalteinheit (2) unter Verwendung eines oder mehrerer Dampfspalteinsatzströme (g, n, I, r) einen Dampfspaltproduktstrom (s) zu erzeugen, **dadurch gekennzeichnet, dass** aus dem Katalyseproduktstrom (b) oder einem Teil hiervon zumindest der überwiegende Teil der Kohlenwasserstoffe mit mehr als vier und/oder weniger als drei Kohlenstoffatomen und des Isobutens entfernt wird und ein oder mehrere jeweils verbleibende Ströme (g, n, l, r) als der oder die Dampfspalteinsatzströme (g, n, l, r) verwendet werden.

2. Verfahren nach Anspruch 1, bei dem zunächst der überwiegende Teil der Kohlenwasserstoffe mit mehr als vier und/oder weniger als drei Kohlenstoffatomen unter Verbleib eines C4-Teilstroms (c) abgetrennt wird und anschließend der überwiegende Teil des Isobutens aus dem C4-Teilstrom (c) entfernt wird.

3. Verfahren nach Anspruch 2, bei dem das Entfernen des überwiegenden Teils des Isobutens die zumindest teilweise Umsetzung des Isobutens und die Abtrennung zumindest eines Teils des dabei gebildeten Umsetzungsprodukts umfasst.

4. Verfahren nach Anspruch 3, bei dem zumindest ein Teil eines nach dem Entfernen des überwiegenden Teils des Isobutens verbleibenden Stroms (g) als der oder einer der Dampfspalteinsatzströme (g) verwendet wird.

5. Verfahren nach Anspruch 2, bei dem das Entfernen des überwiegenden Teils des Isobutens seine destillative Abtrennung umfasst.

6. Verfahren nach Anspruch 5, bei dem vor der destillativen Abtrennung des überwiegenden Teils des Isobutens der überwiegende Teil des in dem C4-Teilstrom (c) enthaltenen 1-Butens zu 2-Buten isomerisiert wird.

7. Verfahren nach einem der Ansprüche 2 bis 6, bei dem aus zumindest einem Teil eines nach dem Entfernen des überwiegenden Teils des Isobutens verbleibenden Stroms (g, h) ein überwiegend n-Butan und 2-Buten enthaltender Strom (n, r) gebildet und als der Dampfspalteinsatzstrom (n, r) oder einer der Dampfspalteinsatzströme (n, r) verwendet wird.

8. Verfahren nach einem der Ansprüche 2 bis 7, bei dem ein Strom (I) gebildet wird, der überwiegend Isobutan enthält und als der Dampfspalteinsatzstrom (n, r) oder einer der Dampfspalteinsatzströme (n, r) verwendet wird.

9. Verfahren nach einem der Ansprüche 2 bis 8, bei dem der Dampfspaltproduktstrom (s) Kohlenwasserstoffe mit vier Kohlenstoffatomen, darunter Butadien, sowie Kohlenwasserstoffe mit mehr als vier und/oder weniger als drei Kohlenstoffatomen enthält.

10. Verfahren nach Anspruch 9, bei dem der überwiegende Teil des Butadiens und der Kohlenwasserstoffe mit mehr als vier und/oder weniger als drei Kohlenstoffatomen aus dem Dampfspaltproduktstrom (s) unter Erhalt eines butadienarmen Reststroms (x), der überwiegend Kohlenwasserstoffe mit vier Kohlenstoffatomen enthält, abgetrennt wird.

11. Verfahren nach einem der Ansprüche 2 bis 10, bei dem zumindest ein Teil des butadienarmen Reststroms mit zumindest einem Teil des C4-Teilstroms (c) vereinigt wird.

12. Anlage (100, 200, 300) zur Herstellung von Kohlenwasserstoffen, mit einer Katalyseeinheit (1), die dazu eingerichtet ist, unter Verwendung eines oder mehrerer, Oxygenate und/oder Olefine enthaltender Katalyseeinsatzströme (a) einen n-Butan, Isobutan, 1-Buten, 2-Buten, Isobuten und Kohlenwasserstoffe mit mehr als vier und/oder weniger als drei Kohlenstoffatomen enthaltenden Katalyseproduktstrom (b) zu erzeugen, und mit einer Dampfspalteinheit (2), die dazu eingerichtet ist, unter Verwendung eines oder mehrerer Dampfspalteinsatzströme (g, n, l, r) einen Dampfspaltproduktstrom (s) zu erzeugen, **dadurch gekennzeichnet, dass** Mittel vorgesehen sind, die dazu eingerichtet sind, aus dem Katalyseproduktstrom (b) oder einem Teil hiervon zumindest den überwiegenden Teil der Kohlenwasserstoffe mit mehr als vier und/oder weniger als drei Kohlenstoffatomen und des Isobutens zu entfernen und einen oder mehrere jeweils verbleibende Ströme (g, n, r) als den oder die Dampfspalteinsatzströme (g, n, I, r) zu verwenden.

13. Anlage (100, 200, 300) nach Anspruch 12, mit Mitteln, die zur Durchführung eines Verfahrens nach einem der Ansprüche 1 bis 11 eingerichtet sind.
